Europäisches Patentamt

(19) European Patent Office

Office européen des brevets

(11) Publication number: **0 018 729**
**A1**

(12) # EUROPEAN PATENT APPLICATION

(21) Application number: **80301071.9**

(22) Date of filing: **03.04.80**

(51) Int. Cl.³: **C 07 C 51/215**
**C 07 B 3/00, C 07 C 27/12**
**C 07 C 29/50, C 07 C 45/33**
**C 07 C 53/08**

(30) Priority: **06.04.79 GB 7912182**

(43) Date of publication of application:
**12.11.80 Bulletin 80/23**

(84) Designated Contracting States:
**BE DE FR GB IT NL**

(71) Applicant: **BP Chemicals Limited**
**Britannic House Moor Lane**
**London, EC2Y 9BU(GB)**

(72) Inventor: **Bartlett, John Stanley**
**BP Chemicals Limited Saltend**
**Hedon Hull, HU12 8DS(GB)**

(72) Inventor: **Pennington, John**
**BP Chemicals Limited Saltend**
**Hedon Hull, HU12 8DS(GB)**

(72) Inventor: **Hudson, Barry**
**BP Chemicals Limited Saltend**
**Hedon Hull, HU12 8DS(GB)**

(74) Representative: **Harry, John et al,**
**BP TRADING LIMITED Patents & Licensing Division**
**Chertsey Road**
**Sunbury-on-Thames Middlesex TW16 7LN(GB)**

(54) **Continuous process for the production of oxygen-containing organic compounds including a substantial proportion of acetic acid, and products thus prepared.**

(57) Oxygen-containing organic compounds including a substantial proportion of acetic acid are produced continuously by liquid phase oxidation of a $C_3$ to $C_7$ saturated hydrocarbon, or mixture of such hydrocarbons, at a temperature in the range 70 to 150°C in the presence of an essentially inert reaction medium and a soluble cobalt catalyst, the total volume of the liquid in the reaction zone being maintained substantially constant. Improvements in this process result from introducing into the reaction zone the cobalt catalyst dissolved in essentially inert reaction medium at a rate exceeding 0.2 kg/hour, preferably exceeding 0.3 kg/hour per litre of reaction mixture.

.TITLE MODiFIED,
see front page

1

CONTINUOUS PROCESS FOR THE PRODUCTION OF OXYGEN
-CONTAINING ORGANIC COMPOUNDS INCLUDING A SUBSTANTIAL
PROPORTION OF ACETIC ACID

The present invention relates to a continuous process for the liquid phase oxidation of saturated aliphatic hydrocarbons containing 3 to 7 carbon atoms, in the presence of a cobalt compound soluble in the reaction medium, to produce oxygen-containing organic compounds including a substantial proportion of acetic acid and in particular to a continuous process for the oxidation of butane to acetic acid.

Processes for the low-temperature cobalt-catalysed oxidation of lower aliphatic hydrocarbons have been known for some time. Thus British Patent Specification No 709,674 describes and claims a process for the partial oxidation of a liquid hydrocarbon, containing less than 6 carbon atoms, in the liquid phase in a solvent medium containing an oxidation catalyst by means of gaseous oxygen wherein the process is carried out under such conditions that water formed as a·product of the oxidation is removed from the reaction mixture substantially as it is formed. In a preferred form of the invention the process is carried out continuously with a portion of the reaction mixture being permitted to distil over during the course of the oxidation, the distillate is condensed and allowed to separate into an aqueous phase and a hydrocarbon phase, and the hydrocarbon phase is returned to the reaction mixture without any intermediate treatment for the removal of oxygen-containing compounds contained therein.

Furthermore British Patent Specification No 1266678 describes and claims a process for converting butane to a product predominating in acetic acid comprising, at a temperature within the range

1

from 66° to 177°C, contacting butane with a gas containing molecular oxygen in the presence of an inert reaction medium and of a catalyst consisting of a cobalt compound soluble in the reaction medium, the cobalt compound being present in an amount calculated as cobalt within the range from 0.4 to 25 per cent by weight based on the reaction medium. The reason for the high catalyst concentrations is stated to be that a substantially greater efficiency of conversion of butane to acetic acid is, surprisingly, achieved without the necessity of removing water as it is formed.

We have now found that the standing water concentration in the reactormay be controlled by the use of higher catalyst solution feed/removal rates than hithertofore and that such a mode of operation can lead to other unforeseen advantages.

Thus according to the present invention there is provided a continuous process for the production of oxygen-containing organic compounds including a substantial proportion of acetic acid by continuously feeding to a reaction zone, maintained at a temperature in the range 70 to 150°C and a pressure sufficient to maintain the reactants essentially in the liquid phase, a saturated aliphatic hydrocarbon containing from 3 to 7 carbon atoms or a mixture thereof, a molecular oxygen-containing gas, an essentially inert reaction medium and a soluble cobalt catalyst and recovering the oxygen-containing organic compounds therefrom by withdrawing gas-free liquid from the reactor at a rate such as to maintain the total volume of liquid in the reaction zone substantially constant and separating by distillation into residual hydrocarbon, a fraction containing the bulk of the oxygenated organic products and a fraction comprising inert reaction medium with the cobalt catalyst dissolved therein, the cobalt catalyst being present in the reaction zone in an amount, calculated as cobalt, in the range of from 0.1 to 10% by weight, based on the weight of reaction medium, characterised in that the cobalt catalyst dissolved in essentially inert reaction medium is introduced continuously into the reaction zone at a rate exceeding 0.2 kg/hour per litre of reaction mixture.

The reaction temperature is preferably in the range 90 to 140°C, and a reaction pressure in the range 10 to 100 bar absolute is usually found to maintain the reactants in the liquid phase. Reaction time is not critical being dependent merely upon the extent of conversion required. Thus the reaction period may be in the range of from one minute to 20 hours, preferably from 10 minutes to 3 hours.

Whilst any saturated aliphatic hydrocarbon containing 3 to 7 carbon atoms or mixture thereof may be employed in the process of the present invention, the preferred hydrocarbon is butane. The butane is preferably used in the form of n-butane, but may contain isobutane and minor amounts of other saturated hydrocarbons. Although isobutane may be present in greater proportions, it is preferred that the n-butane contain not more than 40% w/w isobutane.

The molecular oxygen-containing gas may be substantially pure oxygen or may be any gas mixture containing molecular oxygen, but is preferably air. Air is preferred as oxidant because the likelihood of its forming explosive mixtures in the unreacted gases, either at the top of the reactor or in the overhead gas withdrawal system, can be prevented far more easily than when using substantially pure oxygen or gas mixtures containing higher concentrations of oxygen than air. Alternatively, substantially pure oxygen may be introduced into the reaction mixture together with recycled (or recirculated) off gases from the oxidation process as a means of reducing the likelihood of forming explosive mixtures in the unreacted gases.

The essentially inert reaction medium may be any inert material in which the cobalt catalyst is soluble, but is preferably a lower fatty acid having from two to four carbon atoms, such as acetic acid, propionic acid and normal butyric acid. When butane is the saturated aliphatic hydrocarbon, it is preferred to employ acetic acid, since it is the desired product, and complicated and unnecessary separation procedures are thereby avoided. Other inert reaction media such as benzene, chlorobenzene, phenyl benzoate or benzoic acid may be used. The amount of inert reaction medium employed is not critical provided that a substantially homogeneous reaction medium is maintained throughout the reaction. The weight ratio of inert reaction medium to saturated aliphatic hydrocarbon in the reaction zone may be in the range 1:10 to 100:1.

The oxygenated products resulting from the oxidation of $C_3$ to $C_7$ saturated aliphatic hydrocarbons comprise carboxylic acids in substantial proportions and minor proportions of ketones, esters and oxides of carbon, eg carbon monoxide and carbon dioxide. Thus the oxidation of n-butane results in a product comprising predominantly acetic acid, together with minor amounts of propionic acid and butyric acid, methyl ethyl ketone, sec-butyl acetate, ethyl acetate, methyl acetate, acetone, succinic acid, water, carbon monoxide, carbon dioxide and higher boiling products. Compared with other hydrocarbon oxidation routes to acetic acid, in the oxidation of butane by the process of the present invention the quantity of carbon monoxide, being less than 2% of the acetic acid made, is exceptionally low.

In general the rate at which gas-free liquid is removed from the reactor will be greater than the rate of introduction of the cobalt catalyst dissolved in essentially inert reaction medium into the reactor in order to take account of the products formed in the reaction.

Although the process of the invention is applicable to processes in which the catalyst solution is used on a once-through basis, ie it is discarded after separation from the oxidation products, it is particularly applicable to oxidation processes in which the fraction comprising inert reaction medium with the cobalt catalyst dissolved therein, after separation from the oxidation products, is recycled to the oxidation zone.

Whilst for once-through operation the soluble cobalt catalyst may be any soluble cobaltous or cobaltic compound, for operation in a manner involving continuous recycle of inert reaction medium with the cobalt catalyst dissolved therein not less than 5 and not more than 90, preferably not less than 5 and not more than 50%, of the cobalt catalyst should be introduced into the reaction zone in the +3 oxidation state, ie as a cobaltic compound. The cobalt catalyst containing cobalt partially in the +3 oxidation state may be prepared from the corresponding compound of cobalt in the +2 oxidation state by any means known in the art. Some such suitable means are by co-oxidation in the presence of acetaldehyde, paraldehyde or methylethyl ketone, by treatment with ozone, or by electrochemical oxidation. Compounds of cobalt in the +2 oxidation state suitable for use as the soluble cobalt catalyst in once-through operation or for oxidation to the corresponding compound of cobalt in the +3 oxidation state include cobaltous chloride, sulphate, nitrate, acetate, propionate, butyrate, isovalerate, benzoate, toluate, terephthalate, naphthenate, salicylate, phthalocyanine or acetylacetonate, of which cobaltous acetate is preferred. The cobaltous compound may have been recovered from an earlier oxidation reaction product. The necessary amount of cobalt in the +3 oxidation state in the catalyst may be achieved by partial oxidation of the corresponding compound of cobalt in the +2 oxidation state or simply by mixing a compound of cobalt in the +2 oxidation state with a compound of cobalt in the +3 oxidation state in the desired proportions. Preferably the cobalt compound is present in the reaction zone in an amount calculated as cobalt, in the range of from 0.2 to 5.0% by weight, based on the weight of reaction medium.

Certain of the products resulting from the oxidation of $C_3$ to $C_7$ saturated aliphatic hydrocarbons are capable of reducing cobalt in the +3 oxidation state to cobalt in the +2 oxidation state. The reduction occurs particularly rapidly under the conditions of elevated temperature prevailing during its separation by distillation, and unless measures are taken to maintain the proportion of the cobalt catalyst in the +3 oxidation state in the recycle stream to the oxidation zone within the range 5 to 90, preferably 5 to 50% the oxidation rate may progressively decrease to a point where reaction ceases. Furthermore, it has been noted that when the cobalt catalyst solution is recovered from the reaction products with substantially all the cobalt in the +2 oxidation state, partial deposition as insoluble Co (+2) compounds occurs, leading to rapid fouling of heat-exchanger surfaces and frequent blockages in pipes, pumps etc used for re-introducing the catalysts into the reaction zone, whereas when the catalyst recycle stream contains Co (+3) the problem of solids deposition is very much reduced, thereby contributing to the extended operability of the continuous oxidation process.

It is therefore preferred to separate the cobalt catalyst as a solution in inert reaction medium from the water and oxygen-containing organic compounds with which it is mixed by feeding the mixture to a distillation zone wherein a catalyst solution fraction comprising inert reaction medium and cobalt of which from 5 to 90%, preferably from 5 to 50%, is in the +3 oxidation state for recycle is separated as a base product from a distillate fraction comprising oxygen-containing organic compounds and water, the residence time of the catalyst solution in the distillation zone being so adjusted that it is the minimum consistent with substantially complete separation of the fractions.

The rate of chemical reduction of cobalt in the +3 oxidation state to cobalt in the +2 oxidation state is directly dependent on the temperature, and as a consequence it follows that the shorter the time for which the cobalt catalyst is exposed to the elevated temperature required to separate the reaction products from the catalyst-containing inert reaction medium the less will be the reduction of the cobalt (+3) component of the catalyst to the Co (+2) component.

The residence time of the catalyst solution in the distillation zone is suitably less than 20 minutes, preferably less than 10 minutes, when the separation of the fractions is carried out by distillation at atmospheric pressure.

Residence times consistent with substantially complete separation of the fractions may be achieved by employing as the distillation zone either (a) a packed column having as a reboiler a falling film evaporator, or (b) a shallow tray column, eg of the type used for vacuum distillations, in combination with a falling film evaporator, or (c) a packed column equipped with a reboiler having a volume sufficiently low to limit the residence time, or (d) a shallow tray column in combination with a reboiler of the type described in (c).

In addition, the temperature required to separate the catalyst solution fraction from the distillate fraction may be reduced by maintaining the distillation zone under reduced pressure.

The recovered fraction comprising inert reaction medium and cobalt in the +3 oxidation state may contain, in addition, minor amounts of "heavy ends". Because of their higher boiling point, the "heavy ends" may tend to build up in the catalyst solution recycle stream. For this reason, it may be desirable to remove the heavy ends by cooling all or part of the catalyst solution recycle stream and subjecting it to settling and/or filtration prior to recycle to the reaction oxidation zone.

The crude product may be separated by feeding the product to a distillation column, wherein a fraction comprising water, methyl ethyl ketone, sec-butyl acetate and minor amounts of alcohols and other volatile ketones and esters, which products, not including water, are collectively hereinafter referred to as "light ends", is taken off as an overhead fraction and condensed, thereby forming a water-rich phase and a ketone/ester-rich phase, said water-rich phase being separated and returned wholly or partially to the column at a point near the top thereof and the ketone/ester-rich phase being removed, and passing the base product comprising acetic acid, water and carboxylic acid impurities to a second distillation column wherein water is removed overhead as an azeotrope leaving a base product comprising substantially anhydrous carboxylic acids. By passing the base product to a third distillation column, acetic acid may be separated overhead from the other high-boiling carboxylic acids as a substantially pure product. The ketone/ester-rich phase removed from the first column may be further separated into substantially pure methyl ethyl ketone and sec-butyl acetate products if so-desired, or may be recycled to the reactor, though their presence in the reactor is not necessary to initiate the oxidation reaction.

Alternatively, the condensate from the first distillation column may be separated into a water-rich phase and a ketone/ester-rich phase, the water-rich phase being withdrawn and the ketone/ester-rich phase being returned at least in part to the column, and a base product comprising substantially anhydrous acetic acid and minor amounts of other higher-boiling carboxylic acids passed to a second distillation column wherein substantially pure acetic acid is separated from higher-boiling carboxylic acids. Any ketone/ester rich phase removed from the distillation column may be separated to isolate methyl ethyl ketone and sec-butyl acetate or may be returned directly to the reactor.

The cobalt catalyst, dissolved in essentially inert reaction medium, is preferably introduced continuously into the reaction zone at a rate exceeding 0.3 kg/hour per litre of reaction mixture.

The reaction zone is preferably a vessel either fabricated in or lined, on at least that part of the internal surface thereof in contact with the liquid reaction medium, with a substantially chromium, free material, particularly when the catalyst solution is recycled to the reaction zone. Preferably the chromium-free material is titanium. On the other hand, for once-through operation the reaction zone may be fabricated in stainless steel.

Using the process of the present invention, the conversion of oxygen may be increased at a constant feed-rate of the molecular oxygen-containing gas. The attainment of lower oxygen contents in the effluent gases provides an additional margin of safety with regard to minimising the possibility of forming explosive mixtures. Additionally, the more efficient use of the oxygen introduced into the reaction zone reduces costs associated with the compression of the molecular oxygen-containing gas to reaction pressure. The rate of oxygen consumption may be increased by simultaneously increasing the feed rate of the molecular oxygen-containing gas, without a significant reduction in oxygen conversion. Hence, higher reactor productivities are achieveable without incurring potential safety problems. Using higher catalyst solution feed/recycle rates, in general oxygen conversion and consumption rates show desirable increases in sensitivity to such parameters as temperature and hydrocarbon concentrations.

The invention will now be illustrated by the following Examples.

Comparison Test

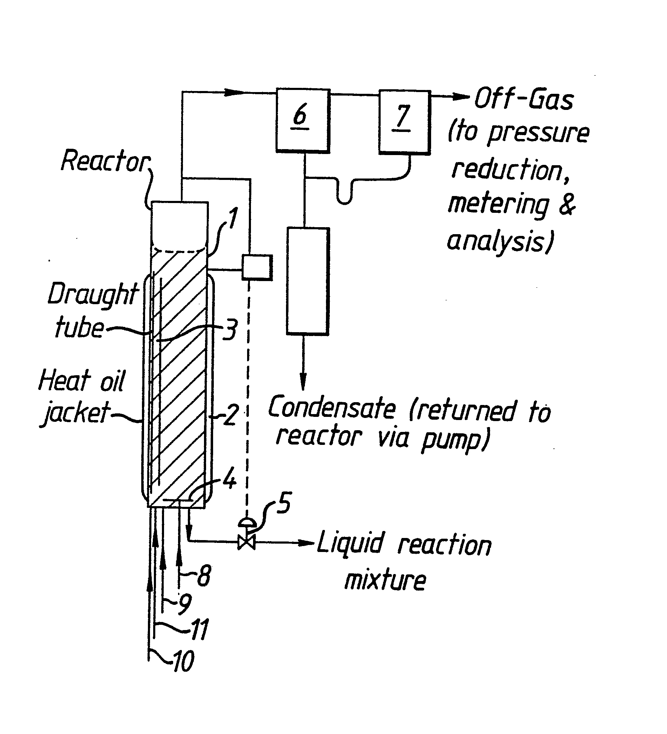

The reaction vessel used in the test is illustrated in the Figure of the accompanying drawing. With reference to the Figure, the numeral 1 denotes a reaction vessel, fabricated in titanium, approximately

9

1.5 m in height and 10 cm inner diameter. 2 is a heating oil jacket; 3 is a 'draught' tube of segmental cross-section, its purpose being to promote circulation of the reactor contents; 4 is an air distributor; 5 is a level-controlled liquid release valve; 6 is a water-cooled condenser; 7 is a refrigerated brine-cooled condenser; 8 is an air inlet pipe; 9 is a butane inlet pipe; 10 is a condensate return pipe and 11 is a catalyst inlet pipe also functioning as a "light ends" return pipe when operating under "light ends" recycle conditions. The volume of aerated reaction mixture, ie up to the pipe to the level controller, and excluding the volume of the draught tube, is approximately 9 litres.

Preparation of catalyst solution

Approximately 5 1 of acetic acid was charged to a glass vessel equipped with a gas inlet pipe and stirrer. Cobaltous acetate was dissolved in the acetic acid to provide a solution containing about 1% cobalt by weight. Oxygen at a rate of about 7 1/h was passed via an OZONO (Registered Trade Mark) air conditioning unit into the stirred vessel at ambient temperature for at least 6 h, thereby converting at least 80% of the cobalt acetate in the solution into the Co (+3) oxidation state. Ozone in the effluent gas was destroyed by passage through vessels containing an aqueous solution of potassium iodide.

Comparison Test

Using the apparatus illustrated in the Figure, the reaction vessel 1 was charged with 8 litres of glacial acetic acid and was pressurised to about 35 bar (absolute). A small nitrogen flow was then established through the pipe 8. Approximately 1 kg of butane was charged rapidly to the reactor through the line 9 and the butane feed rate was then adjusted to ca 0.6 kg/h. The oil-heating system was switched on, and a cobalt catalyst solution, comprising 2.2% w/w cobalt, 68% as Co (+3), in acetic acid containing ca 4% w/w water, was introduced through line 10 at a rate of ca 1.2 1/h.

When the reactor temperature reached 60°C, air was substituted for the nitrogen stream. The oxidation reaction commenced within 15 minutes of attaining a reaction temperature of 120°C. The reaction temperature was stabilised at 125°C, and the air and butane

10

feed rates were adjusted to provide an oxygen content in the off-gas of ca 4% v/v and a (net) acetic acid production rate of approximately 450 g/h.

Liquid reaction mixture withdrawn from the reactor through the valve 5, at a rate such as to maintain the total volume of liquid in the reaction zone substantially constant, was passed to a distillation column (approximately 3.5 mm in diameter and 1.8 m in height. Packed with 6 mm ceramic Raschig rings), fitted with a specially designed low residence time reboiler fabricated in titanium, to provide a recovered catalyst solution containing cobalt, from 5 to 90% of which was in the +3 oxidation state. Recycle of this catalyst solution through line 10 commenced approximately 3 hours after start-up. The catalyst solution recycle rate was approximately 1.25 kg/h, corresponding to 0.14 kg/h per litre of reaction mixture.

"Light ends" were separated from the reaction products in a 20-plate Oldershaw column of approximately 50 mm diameter. The feed mixture was introduced near to the midpoint of the column. The distillate separated into two phases and the whole of the lower (aqueous) phase was returned to the top of the column, while the upper (ketone/ester) phase was withdrawn. The base product, withdrawn from the reboiler under liquid level control, comprised essentially all the carboxylic acids and the greatest part of the water, with no ketones or esters detectable by gas/liquid chromatography. The ketone/ester phase withdrawn comprised the light ends having the approximate composition by weight: 70% MEK, 20% sec-butyl acetate, 5% ethyl acetate, 2.5% methyl acetate and 1.5% acetone. Additionally the "light ends" contained about 6% water. The "light ends" were thereafter recycled to the reaction vessel.

The continuous oxidation process was operated for a period of approximately 16 days with occasional interruptions, during which period no significant interruption of the oxidation reaction occurred over about 7 consecutive days. During the period of operation reaction conditions were varied to investigate the effects of individual reaction parameters. Results obtained over 8 hours towards the end of this period are given in the Table.

0018729

## TABLE

|                                                                                      | "Light-ends" Recycled |
| ------------------------------------------------------------------------------------ | --------------------- |
| Reaction Temperature (°C) <br> Reaction Pressure (bar gauge) <br> Butane Feedrate (kg/h) <br> Air Feedrate (m³/h at STP) | 130.5 <br> 48.3 <br> ca. 1.5 <br> ca. 4.6 |
| Cobalt Concentration in Reactor <br> Product (Butane Free) (% w/v) <br> Proportion as Co(+3) (%) <br><br> Cobalt Concentration in Recycled <br> Catalyst Solution (% w/v) <br> Proportion as Co(+3) (%) | 0.28 <br> 26 <br><br> 0.86 <br> 10 |
| Oxygen Consumption Rate (Kg/h) <br> Acetic Acid Production <br> Rate (kg/h) | 1.15 <br><br> 1.07 |
| Weight Selectivities <br> (g/100 g butane consumed) <br><br> Acetic Acid <br> Propionic Acid <br> Butyric Acid <br> Carbon Dioxide <br> Carbon Monoxide | <br><br> 169 <br> 3 <br> 2 <br> 39 <br> 2 |

For the period over which the results presented in the Table were collected the concentration of n-butane in the reaction mixture was 11 to 12% w/w and the resulting conversion of oxygen was 82%.

An increase in the cobalt concentration (to ca 0.9 to 1.0% w/v in the reaction mixture), at a marginally lower air rate, led to oxygen consumption rates of 1.05 to 1.1 kg/h at an oxygen conversion of approximately 84%. Alternatively, an increase in n-butane concentration to over 15% w/w led to similar very small changes in oxygen consumption rate and conversion.

Operation at higher air feed rates for short periods led to increased oxygen concentrations in the reactor off-gases, in a manner indicative of little increase in oxygen consumption rate.

This is not an example according to the present invention, because the catalyst solution feed/recycle rate was less than 0.2 kg/h per litre of reaction mixture. It is included for the purpose of comparison only.

Example 1

Owing to the limited capacity of the catalyst recovery/recycle system described in the Comparison Test, a fresh solution of cobaltous acetate was substituted for the catalyst recycle stream. The apparatus and procedure described in the Comparison Test were again used, except that the liquid reaction mixture withdrawn from the reactor through line 5 was not passed to a distillation column fitted with a low residence time reboiler. Instead, it was subjected to flash distillation. The distillate so-obtained was fed to the "light ends" separation column, from which the "light ends" were recycled to the reactor. The separated catalyst solution was discarded.

The concentration of cobalt in the solution introduced to the reactor was adjusted to provide a cobalt concentration of 0.5 to 0.7% w/v in the reaction mixture. The reaction pressure was 48.3 bar (gauge), the reaction temperature was approximately 130°C and the air feed rate was restricted to ca $4m^3$/h (referred to STP).

At an n-butane concentration of 10 to 13%, stepwise increases in the catalyst solution feed rate from 0.14 kg/1.h to 0.32 kg/1.h led to increases in oxygen conversion from approximately 87.5% to 91%, with corresponding small increases in oxygen consumption rate. A further increase in the catalyst solution feed rate of 0.6 kg/1.h, together with an increase in n-butane concentration to 16 to 17%, led to an oxygen conversion of 93.5% at an oxygen consumption rate of 1.1 kg/h.

Hence, by the use of a higher catalyst solution feed rate, an oxygen consumption rate similar to the value obtained in the Comparison Test was achieved at a significantly higher oxygen utilisation efficiency.

Example 2

This Example followed that described in Example 1 without interruption.

With the catalyst solution feed rate at approximately 0.6 kg/1.h (with the temperature and pressure maintained at the values obtaining in Example 1 and an n-butane concentration of about 14% w/w), the air feed rate was increased to ca 6.5 $m^3$/h (referred to STP). The quantity of oxygen consumed rose to over 1.5 kg/h at an oxygen conversion of 81%.

Subsequently, increases in the catalyst solution feed rate to approximately 1.1 kg/1.h, the air feed rate to ca 8 $m^3$/h and the temperature to 135°C provided an oxygen consumption rate of over 1.8 kg/h at an oxygen conversion of 78%.

Hence, the use of higher catalyst solution feed rates provides a means of achieving very much higher oxygen consumption rates, albeit at the expense of small reductions in oxygen conversions when compared with the Comparison Test.

Example 3

The reactor described in the Comparison Test was started up and operated in a similar manner to that described in the Test except that the reaction temperature and pressure were adjusted to 135°C and 55 bar (gauge) respectively.

The liquid reaction products were subjected to flash distillation in the manner described in Example 1.

The residue solutions from this flash distillation step were passed into a catalyst solution recovery column consisting of 10 Oldershaw plates to 50 mm diameter. The reboiler on this column was now too small to provide the vapour flow rate necessary to effect separation of water, and additional dry acetic acid vapour was introduced immediately above the reboiler. Catalyst solution, wherein at least 5% of the cobalt was in the +3 oxidation state, was withdrawn from the reboiler and recycled to the reaction vessel at a rate of approximately 0.4 kg/l.h.

The distillate fractions from the flash distillation and catalyst solution recovery stages were combined and passed to the column as described in the Comparison Test for partial recovery and recycle of "light ends".

With an air feed rate of 5.3 $m^3$/h (referred to STP) and an n-butane concentration of approximately 13% w/w, the oxygen consumption rate was over 1.3 kg/h at an oxygen conversion of approximately 85%. The net production rate of acetic acid was approximately 1.2 kg/h.

## Claims

1. A continuous process for the production of oxygen-containing organic compounds including a substantial proportion of acetic acid by continuously feeding to a reaction zone, maintained at a temperature in the range 70 to 150°C and a pressure sufficient to maintain the reactants essentially in the liquid phase, a saturated aliphatic hydrocarbon containing from 3 to 7 carbon atoms or a mixture thereof, a molecular oxygen-containing gas, an essentially inert reaction medium and a soluble cobalt catalyst and recovering the oxygen-containing organic compounds therefrom by withdrawing gas-free liquid from the reactor at a rate such as to maintain the total volume of liquid in the reaction zone substantially constant and separating by distillation into residual hydrocarbon, a fraction containing the bulk of the oxygenated organic products and a fraction comprising inert reaction medium with the cobalt catalyst dissolved therein, the cobalt catalyst being present in the reaction zone in an amount, calculated as cobalt, in the range of from 0.1 to 10% by weight, based on the weight of reaction medium, characterised in that the cobalt catalyst dissolved in essentially inert reaction medium is introduced continuously into the reaction zone at a rate exceeding 0.2 kg/hour per litre of reaction mixture.

2. A process according to claim 1 wherein the saturated hydrocarbon comprises n-butane.

3. A process according to either one of the preceding claims wherein the oxygen-containing gas is air.

4. A process according to any one of the preceding claims wherein the essentially inert reaction medium is a lower fatty acid having from 2 to 4 carbon atoms.

5. A process according to either claim 2 or claim 4 wherein the essentially inert reaction medium is acetic acid.

6. A process according to any one of the preceding claims wherein the soluble cobalt catalyst is cobaltous acetate.

7. A process according to any one of claims 1 to 5 wherein not less than 5 and not more than 90% of the cobalt catalyst is introduced into the reaction zone in the +3 oxidation state.

8. A process according to any one of the preceding claims wherein the cobalt catalyst is separated as a solution in inert reaction medium from the water and oxygen-containing compounds with which it is mixed by feeding the mixture to a distillation zone wherein a catalyst solution fraction comprising inert reaction medium and cobalt of which from 5 to 90% is in the +3 oxidation state is separated as a base product from a distillate fraction comprising oxygen-containing organic compounds and water, the residence time of the catalyst solution in the distillation zone being so adjusted that it is the minimum consistent with substantially complete separation of the fractions.

9. A process according to any one of the preceding claims wherein the inert reaction medium with the cobalt catalyst dissolved therein, after separation from the oxidation products, is recycled to the oxidation zone.

10. A process according to any one of the preceding claims wherein the cobalt catalyst dissolved in essentially inert reaction medium is introduced continuously into the reaction zone at a rate exceeding 0.3 kg/hour per litre of reaction mixture.

11. A process according to any one of the preceding claims wherein the reaction zone is a vessel fabricated or lined, on at least that part of the internal surface thereof in contact with the liquid reaction medium, with a substantially chromium free material.

12. A process substantially as hereinbefore described with reference to Examples 1 to 3.

13. Oxygen-containing organic compounds including a substantial proportion of acetic acid whenever prepared by a process as claimed in any one of the preceding claims.

Reactor

Draught
tube

Heat oil
jacket

1
3
2
4
5
8
9
11
10

6  7  → Off-Gas
(to pressure
reduction,
metering &
analysis)

Condensate (returned to
reactor via pump)

→ Liquid reaction
mixture

0018729

| | **DOCUMENTS CONSIDERED TO·BE RELEVANT** | | CLASSIFICATION OF THE APPLICATION (Int. Cl.³) |
|---|---|---|---|
| Category | Citation of document with indication, where appropriate, of relevant passages | Relevant to claim | |
| X | US - A - 4 131 741 (J.S. BARTLETT et al.)  *  the whole document  *  -- | 1-9, 12,13 | C 07 C 51/215<br>C 07 B 3/00<br>C 07 C 27/12<br>C 07 C 29/50<br>C 07 C 45/33<br>C 07 C 53/08 |
| | US - A - 4 032 570 (J.G.D. SCHULZ et al.)  *  claims 1 to 6  *  -- | 1-5 | |
| | FR - A1 - 2 329 627 (BP CHEMICALS)  *  claims 1, 3, 5, 8, 10 to 13, 15  *  -- | 1-9, 12,13 | **TECHNICAL FIELDS SEARCHED (Int.Cl.³)**<br><br>C 07 B 3/00<br>C 07 C 27/12<br>C 07 C 29/00<br>C 07 C 29/50<br>C 07 C 45/32<br>C 07 C 45/33<br>C 07 C 51/21<br>C 07 C 51/215<br>C 07 C 51/225<br>C 07 C 53/08 |
| A,P | GB - A - 1 553 820 (BP CHEMICALS)  *  claims 1, 14 to 17, 20, 22  *  ---- | 1-5 | |

CATEGORY OF
CITED DOCUMENTS

X: particularly relevant
A: technological background
O: non-written disclosure
P: intermediate document
T: theory or principle underlying
   the invention
E: conflicting application
D: document cited in the
   application
L: citation for other reasons

&: member of the same patent
family,
corresponding document

| | | | |
|---|---|---|---|
| The present search report has been drawn up for all claims | | | |
| Place of search<br>Berlin | Date of completion of the search<br>08-07-1980 | Examiner<br>KNAACK | |

EPO Form 1503.1 06.78